# EUROPEAN PATENT APPLICATION

(11) **EP 4 659 749 A1**
(43) Date of publication of application: **10.12.2025**
(21) Application number: 24179877.6
(22) Date of filing: 04.06.2024
(51) Int. Cl.: A61K 31/357, A61P 33/06, A61K 9/00, A61K 9/14

(54) **INTRANASAL ARTESUNATE POWDER FOR THE TREATMENT OF SEVERE MALARIA**

(71) Applicant: SAS Eunovis, 69006 Lyon (FR)
(72) Inventor: PICOT, Stéphane, 38170 Seyssinet-Pariset (FR); BIENVENU, Anne-Lise, 69006 Lyon (FR)
(74) Representative: Plasseraud IP

(57) **Abstract**

The present invention relates to artesunate powder for use in the treatment of severe malaria, wherein artesunate powder is administered intranasally at a dose which is lower than the artesunate dose administered in a standard treatment of severe malaria. This treatment is particularly useful as a pre-referral treatment.

## Description

### Background of the Invention

Malaria is one of the most frequent parasitic infectious diseases in the world. It is caused by a eukaryotic microorganism of the *Plasmodium* genus which is transmitted through the bite of an infected *Anopheles* female mosquito. Malaria is one of the deadliest human infectious diseases, responsible for more than 600,000 deaths in 2022, most of them being young children in sub-Saharan Africa (World Health Organization, World malaria report, 2023, Geneva; Tukwasibwe et al., Cell Mol. Immunol., 2020, 17: 799-806). While a great majority of cases are uncomplicated and successfully treated with oral antimalarials, severe malaria may occur in non-immune patients, including children under five years old living in high transmission endemic regions (World Health Organization, World malaria report, 2022, Geneva; Milner et al., Malari Pathogenesis, Cold Spring Harb Perspect. Med., 2018, 8:a025569; White et al., The Lancet, 2014, 383; 723-735; Cowman et al., Cell, 2016, 167: 610-614). A rapid and optimal antimalarial treatment is critical to improve the outcome of severe cases.

The first-line treatment recommended for severe malaria is intravenous artesunate, a semi-synthetic derivative of artemisinin. However, in rural areas where intravenous treatments are not available, the World Health Organization (WHO) recommends the use of a pre-referral artesunate treatment before addressing the patient to the nearest hospital for appropriated care (WHO, World malaria report, 2021, Geneva; Gomes et al., Lancet, 2009, 373: 557-566). Pre-referral artesunate treatment is a single dose of artesunate administered intrarectally to young children (< 6 years old) at risk of severe malaria when oral route is unavailable (World Health Organization, World malaria report, 2021, Geneva; Gomes et al., Lancet, 2009, 373: 557-566). This pre-referral treatment was demonstrated to reduce the risk of death or permanent disability (Gomes et al., Lancet, 2009, 373: 557-566). Pharmacokinetic studies have shown large interindividual variability with a bioavailability ranging from 11.7% to 54.4% (mean = 25.6%), but a 12h-parasite reduction ratio comparable to intravenous artesunate (Fanello et al., Antimicrob. Agents Chemother., 2021, 65: e02223-20). The rectal route is advantageously non-invasive, usable in unconscious or vomiting patients; allows systemic drug absorption; and avoids at least partially the hepatic first-pass effect (Jannin et al., Adv. Drug Deliv. Rev., 2014, 73: 34-49). However, a negative cultural perception of the rectal route, as well as reports of melting artesunate suppositories under tropical and subtropical temperatures could limit its use (Jannin et al., Adv. Drug Deliv. Rev., 2014, 73: 34-49; Marijon et al., Malar. J., 2014, 13: 501; Bewley et al., BMJ, 2008, 336: 764). Although two artesunate suppositories have now been prequalified by the WHO, there is still little evidence for the implementation of this pre-referral treatment into endemic countries' guidelines for severe malaria management (Signorell et al., PLoS Med., 2023, 20(2):e1004189; Rectal Artesunate Landscaping Assessment Report, Sev. Malar. Obs., 2021, webpage: severemalaria.org/resources/rectal-artesunate-landscaping-assessment-report). Furthermore, artesunate suppositories are not consistently available throughout public health centers, such as for example in Ethiopia (Woldeyohanins et al., Pediatr. Health Med. Ther., 2021, 12: 421-429) and in Kenya (Amboko et al., Trop. Med. Int. Health., 2022, 27: 330-336). More importantly, the WHO recently recommended a moratorium on the implementation of pre-referral artesunate suppository treatment because of the higher case fatality ratio in rectal artesunate recipients reported in a non-randomized, sequential observational study (CARAMAL) conducted in Nigeria, Uganda, and the Democratic Republic of Congo (Brunner et al., BMJ Glob. Health., 2022, 7: e008346). This decision was highly controversial, but it clearly decreased the potential role of the rectal route (Watson et al., BMJ Glob. Health, 2022, 7: e010006; White et al., Trans. R. Soc. Trop. Med. Hyg., 2024, May 25: trae036, online ahead of print).

In this context, there is an urgent need for the development of an alternative artesunate pre-referral treatment.

Intranasal administration has been shown to be an attractive alternative to oral and parenteral routes (Pires et al., J. Pharm. Pharm. Sci., 2009, 12: 288-311; Grassin-Delyle et al., Pharmacol. Ther., 2012, 134: 366-379). The number of drugs administered *via* the nasal route has increased, and include analgesics, anti-migraine agents, cardiovascular medicines, hormones, and anti-inflammatory drugs. The advantages of the nasal route are non-invasiveness, ease of administration, and fast drug absorption (Abd-Elal et al., Drug Deliv., 2016, 23: 3374-3386; Dey et al., Der Pharmacia Sinica, 2011, 2(3): 94-106). The richly vascularized nasal mucosa is effective for drug absorption depending on the regional nasal deposition pattern (Shang et al., Inhal. Toxicol., 2015, 27: 694-705). The respiratory mucosa is the preferential site for systemic drug absorption (Dey et al., Der Pharmacia Sinica, 2011, 2(3): 94-106; Selvaraj et al., Artif. Cells Nanomed. Biotechnol., 2018, 46(8): 2088-2095; Crowe et al., Life Sci., 2918, 195; 44-52), whereas the olfactory mucosa is the preferential site for nose-to-brain drug absorption (Dey et al., Der Pharmacia Sinica, 2011, 2(3): 94-106; 26).

In a preliminary study using a murine model of cerebral malaria (a serious neurological complication of severe malaria), the present Inventors have demonstrated that intranasal delivery of artesunate prevents the development of cerebral malaria (Marijon et al., Malar. J., 2014, 13: 501). They showed that dihydroartemisinin, the main metabolite of artesunate, is recovered in the brain of mice soon after artesunate intranasal administration, which prevents cerebral malaria development. They thus hypothesized that intranasal administration of artesunate for severe malaria treatment would allow both the systemic effects of artesunate, and the nose-to-brain delivery of the drug that would prevent cerebral malaria. Recently, they investigated the capacity of artesunate powder to cross the nasal multilayer epithelial cells without local toxicity, and then to reach the olfactory and respiratory zones of the nose after being sprayed in the nostrils (Kouakou et al., Malar. J., 2022, 21: 291). They used a model of human nasal mucosa to demonstrate that a powder formulation of artesunate had a greater permeation efficiency than the standard artesunate solution. This study also demonstrated that a significant amount of sprayed artesunate (45%) reached the olfactory zone allowing a nose-to-brain delivery that could be of major interest for targeting *Plasmodium* parasites sequestered in the cerebral microvasculature. Almost 20% of the sprayed dose reached the respiratory zone, providing a pathway avoiding the hepatic first-pass effect. The results obtained suggest that artesunate nasal drug administration opens new opportunities in the context of severe malaria.

### Summary of the Invention

The present invention relates to intranasal administration of artesunate powder for the treatment of severe malaria, in particular in the context of pre-referral. In a method of treatment according to the present invention, the dose of artesunate administered intranasally is lower than the dose of artesunate administered in a standard treatment of severe malaria. This contrasts with the considerations of the scientific and medical communities, which are discussing the need of increasing drug doses (Kouakou et al., Int. J. Infec. Dis., 2019, 89: 30-44; Haghiri et al., Clin. Pharmacol. Ther., 2023, 114(6): 1304-1312) in view of the emergence of resistance to artemisinin antimalarials. This resistance poses one of the greatest threats to malaria control and is responsible for increased malaria-related morbidity and mortality (Lubell et al., Malar. J. 2014, 13: 452, Ouji et al., Parasite, 2018, 25: 24; Ishengoma et al., Nat. Med., 2024, April 25, online ahead of print).

Consequently, the present invention relates to artesunate for the use in the treatment of severe malaria in a subject, wherein artesunate is administered as a powder to the subject in need thereof *via* intranasal delivery and wherein artesunate is administered to the subject at a dose which is lower than the dose of artesunate administered in a standard treatment of severe malaria.

In certain embodiments, the treatment of severe malaria is a pre-referral treatment.

In certain embodiments, the treatment of severe malaria comprises prevention of at least one complication of severe malaria, in particular cerebral malaria.

In certain embodiments, the subject is a child, in particular a child of 5 years of age or younger living in a malaria endemic country.

In other embodiments, the subject is a child over 5 years old, an adolescent or a young adult living in a malaria endemic country.

In yet other embodiment the subject is a non-immune child or adult, in particular a pregnant woman, a patient with HIV/AIDS, a non-immune migrant or a non-immune foreign traveler.

In certain embodiments, intranasal delivery of artesunate is performed by intranasal spraying. Preferably, intranasal spraying is performed using a nasal spray device, in particular a pre-filled, ready-to-use nasal spray device. Alternatively, the nasal spray device can be filled just before artesunate administration.

In certain embodiments, the standard treatment of severe malaria is a first-line treatment of severe malaria and comprises:
a) administration of artesunate at a dose of 2.4 mg/kg body weight by intravenous or intramuscular injection to an adult or a child weighing 20 kg or more; or
b) administration of artesunate at a dose of 3 mg/kg body weight by intravenous or intramuscular injection to a child weighing less than 20kg.

In other embodiments, the standard treatment of severe malaria is a pre-referral treatment of severe malaria and comprises:
a) administration of artesunate at a dose of 2.4 mg/kg body weight by injection to an adult or a child weighing 20 kg or more; or
b) administration of artesunate at a dose of 10 mg/kg body weight by rectal administration to a child weighing less than 20kg.

In certain embodiments, the dose of artesunate administered *via* intranasal delivery in a method according to the present invention is lower than 3 mg/kg body weight. The dose of artesunate administered *via* intranasal delivery may be comprised between about 2 mg/kg body weight and about 0.5 mg/kg body weight, in particular comprised between about 1.75 mg/kg body weight and about 0.75 mg/kg body weight, or between about 1.5 mg/kg body weight and about 1 mg/kg body weight.

In another aspect, the present invention relates to a pre-filled, ready-to-use nasal spray device containing artesunate powder for use in the pre-referral treatment of severe malaria in subject, wherein artesunate present in the nasal spray device is in powder form and is administered to the subject at a dose which is lower than the dose of artesunate administered in a standard treatment of severe malaria.

In certain embodiments, the nasal spray device comprises a dose of artesunate which is lower than 3 mg/kg body weight. The dose of artesunate present in the nasal spray device and to be administered *via* intranasal delivery may be comprised between about 2 mg/kg body weight and about 0.5 mg/kg body weight, in particular comprised between about 1.75 mg/kg body weight and about 0.75 mg/kg body weight, or between about 1.5 mg/kg body weight and about 1 mg/kg body weight.

These and other objects, advantages and features of the present invention will become apparent to those of ordinary skill in the art having read the following detailed description of the preferred embodiments.

### Detailed Description of Certain Preferred Embodiments

As mentioned above, the present invention relates to artesunate powder intranasal administration for use in the treatment of severe malaria, wherein artesunate is administered as a single dose that is lower than the dose of artesunate administered in a standard treatment of severe malaria, in particular a standard pre-referral treatment of severe malaria.

### I - Artesunate and Artesunate Powder Formulation

Artesunate displays high and fast antiparasitic properties with a broad specificity of action on the various stages of the *Plasmodium* parasite's life cycle. Its outstanding ability to rapidly kill circulating ring-stage parasite, preventing their maturation and sequestration in deep organs, including the brain, explains its high efficiency to rescue severe malaria (Terkuile et al., Exp. Parasitol., 1993, 76: 85-95; Udomsangpetch et al., J. Infect. Dis., 1996, 173: 691-698; Wilson et al., Antimicrob. Agents Chemother., 2013, 57 : 1455-1467). Artesunate is on the WHO's List of Essential Medicines.

As used herein, the term *"**artesunate**"* has its art understood meaning and refers to a semi-synthetic derivative of artemisinin, which is a naturally occurring sesquiterpene lactone endoperoxide originally isolated from qinghao (sweet wormwood, *Artemisia annua* L.), a plant used for centuries in traditional Chinese medicine. Artesunate is the succinic acid half ester derivative of dihydroartemisinin, which itself may be obtained by sodium borohydride reduction of artemisinin. Artesunate has the molecular formula: C₁₉H₂₈O₈, CAS Number: 88495-63-0, and a molar mass of 384.425 g/mol, and its IUPAC name is 4-oxo-4-[[(1*R*,4*S*,5*R*,8*S*,9*R*,10*S*,12*R*,13*R*)-1,5,9-trimethyl-11,14,15,16-tetraoxa-tetracyclo[10.3.1.0^{4,13}.0^{8,13}]hexadecan-10-yl]oxy]butanoic acid.

Artesunate contains eight chiral centers, of which seven are derived from the starting material artemisinin. Therefore, the term "artesunate", as used herein, encompasses any of the individual enantiomers of artesunate. In particular, the term may refer to just a single enantiomer, or a racemic or non-racemic mixture of the enantiomers.

Artesunate is a non-hygroscopic fine, white to almost white, crystalline powder, which is slightly soluble in water, very soluble in dichloromethane, freely soluble in ethanol and acetone. In the context of the present invention, artesunate is administered intranasally as a powder. As used herein, the term *"**artesunate powder***" refers to artesunate in the form obtained after synthesis from artemisinin, for example following purification. It also refers to micronized and sterilized artesunate powders that are commercially available, such as for example from manufacturer IPCA Laboratories Limited Sejavta, India; Guilin Pharmaceutical Company, China; Amivas, Nassau, USA; and Fosun Pharma, China. Artesunate powder used in the context of the present invention has a purity of at least 97%, for example about 98%, or about 99%, or more than about 99%.

Artesunate powder may be used as such or as an artesunate powder formulation. As used herein, the term *"**artesunate powder formulation**"* refers to a pharmaceutical composition of artesunate comprising a therapeutically effective amount of artesunate powder and at least one pharmaceutically acceptable carrier or excipient such that the final artesunate powder formulation is itself a powder. As used herein, the term *"t**herapeutically effective amount**"* refers to any amount of a therapeutic agent (here: artesunate) that is sufficient to fulfil its intended purpose(s), *e.g.*, a desired biological or medicinal response in a cell, tissue, system, or subject. The term *"**pharmaceutically acceptable carrier or excipient**"* refers to a carrier medium which does not interfere with the effectiveness of the biological activity of the active ingredient (here: artesunate) and which is not excessively toxic to the host at the concentration at which it is administered. The use of such carriers and excipients for pharmaceutically active substances is well known in the art (see for example *"*Remington's Pharmaceutical Sciences", E.W. Martin, 18th Ed., 1990, Mack Publishing Co.: Easton, PA, which is incorporated herein by reference in its entirety). Examples of inert carriers or excipients that can be used for the formulation of a nasal powder drug include, but are not limited to, starches (such as cornstarch), mannitol, lactose, microcrystalline cellulose (MCC), colloidal MCC, chitosan derivative, carboxymethyl chitosan, carboxymethyl cellulose, low-methoxyl pectin, and mucoadhesive agents such as hydroxypropyl methyl cellulose (HPMC), hydroxypropyl cellulose (HPC) and hydroxyethyl cellulose (HEC).

Prior to use, the artesunate powder, or artesunate powder formulation, is preferably stored under appropriate conditions, *e.g*., at room temperature if below 30°C, and away from light. Alternatively, the artesunate powder, or artesunate powder formulation, may be refrigerated (*e.g*., at around 4°C).

It is also contemplated that artesunate powder may be administered after nanoformulation. Nanoformulations for intranasal drug delivery are known in the art. They may increase the concentration of drug administrable in a dose; protect the active ingredient from chemical and/or enzymatic degradation; improve drug stability *in situ;* and promote systemic absorption and/or nose-to-brain absorption. Examples of such nanoformulations include, but are not limited to, solid lipid nanoparticles, and mucoadhesive nanoparticles.

### II - Use of Artesunate Powder in the Treatment of Severe Malaria

The present invention relates to artesunate for use in the treatment of severe malaria in a subject, wherein artesunate is administered as a powder to the subject in need thereof *via* intranasal delivery and wherein the dose of artesunate administered to the subject is lower than the dose of artesunate administered in a standard treatment of severe malaria. The present invention further relates to a method for treating severe malaria in a subject, the method comprising a step of administering intranasally to the subject in need thereof a therapeutically effective amount of artesunate, wherein artesunate is administered as a powder and wherein the dose of artesunate administered to the subject is lower than the dose of artesunate administered in a standard treatment of severe malaria. The present invention also relates to the use of artesunate powder for the manufacture of a medicament for the treatment of severe malaria, wherein the medicament is to be administered intranasally, and wherein the dose of artesunate to be administered to the subject is lower than the dose of artesunate administered in a standard treatment of severe malaria. It is also contemplated that a method of treatment according to the present invention comprises the administration of dihydroartemisinin, the main metabolite of artesunate, instead of artesunate.

The term *"**treatment**"* is used herein to characterize a method or process that is aimed at (1) slowing down or stopping the progression, aggravation or deterioration of the disease, disorder or condition (here: severe malaria); (2) bringing about amelioration of the symptoms of the disease, disorder or condition; (3) delaying or preventing the onset of symptoms or complications of the disease, disorder or condition; or (4) curing the disease, disorder or condition. A treatment may be administered after initiation of the disease, disorder or condition, for a therapeutic action. Alternatively, a treatment may be administered prior to the onset of the disease, disorder or condition or the onset of a complication of the disease, for a prophylactic or preventive action. In this case, the term *"**prevention**"* is used.

### 1 - Indications

As used herein, the term *"**malaria**"* has its art understood meaning and refers to an infectious disease caused by parasites of the genus *Plasmodium,* and which is transmitted through the bite of infected *Anopheles* female mosquitoes. The parasite may belong to the *Plasmodium falciparum, P. vivax, P. ovale, P. malariae, P. knowlesi* or zoonotic species. Malaria typically produces a string of recurrent attacks, or paroxysms, each of which has three stages - chills, followed by fever, and then sweating. Along with chills, the person is likely to have headache, malaise, fatigue, muscular pains, occasional nausea, vomiting, and diarrhea. Within an hour or two, the body temperature rises, and the skin feels hot and dry. Adults with malaria tend to experience chills and fever as well as headache, fatigue, abdominal discomfort, and muscle pain, whereas children tend to have more general symptoms: fever, cough, vomiting, and diarrhea.

According to the present invention, artesunate powder is administered to treat severe malaria. Malaria is classified as either *"**severe**"* or *"**uncomplicated**"* by the WHO. Malaria is deemed severe when any of the following criteria is present, otherwise it is considered uncomplicated:
- Impaired consciousness or coma,
- Prostration (general weakness so that the patient is unable to sit, stand or walk without assistance),
- Failure to feed,
- Multiple convulsions,
- Acidotic breathing,
- Circulatory collapse,
- Clinical jaundice with evidence of another vital organ dysfunction,
- Hemoglobinuria,
- Abnormal spontaneous bleeding, and
- Pulmonary edema.
Laboratory findings can include:
- Hypoglycemia (blood glucose < 2.2 mmol/L or < 40 mg/dL),
- Metabolic acidosis (plasma bicarbonate < 15 mmol/L),
- Severe normocytic anemia (Hb < 5 g/dL),
- Hyperparasitemia (a parasite level in the blood >2%, *i.e.*, 100,000 per µL in low intensity transmission areas, or > 5%, *i.e*., 250,000 per µL in areas of high stable malaria transmission),
- Renal impairment (serum creatinine > 265 µmol/L), and
- Low blood pressure (less than 70 mmHg in adults and 50 mmHg in children).

In children, malaria has a short course, often rapidly progressing to severe malaria. Children with severe malaria are more likely to present with hypoglycemia, seizures, severe anemia, and sudden death, but are much less likely to develop renal failure, pulmonary edema, or jaundice. In the context of the present invention, the term *"**severe malaria**"* is as defined by the WHO (WHO, Trans. R. Soc. Trop. Med. Hyg., 2000, 94(suppl. 1): S1-S90).

Severe malaria cases are medical emergencies since mortality rates are high (10% to 50% or higher depending on medical care).

An intranasal artesunate treatment according to the present invention may slow down or stop the progression of severe malaria; lead to amelioration of at least one symptom of severe malaria; and/or prevent a complication of severe malaria. In some embodiments, a treatment according to the present invention is used to treat or prevent a complication of severe malaria. Examples of complications of severe malaria include, but are not limited to, cerebral malaria, acute kidney failure, pulmonary edema, severe anemia, and severe bleeding.

As indicated above, the present Inventors have shown that artesunate powder exhibits a good permeation efficiency on human nasal mucosa (Kouakou et al., Malar. J., 2022, 21: 291), and measured that a very significant amount of sprayed artesunate (45%) reaches the olfactory zone allowing a nose-to-brain delivery that is of major interest for targeting *Plasmodium* parasites sequestered in the cerebral microvasculature in cerebral malaria. Therefore, in some embodiments, a treatment according to the present invention is used to treat or prevent cerebral malaria. As used herein, the term *"**cerebral malaria**"* refers to a neurological complication of severe malaria defined by the WHO as a severe form of *P. falciparum* malaria that causes cerebral manifestations. Cerebral malaria is characterized by loss of blood-brain-barrier integrity and neurological impairment. The symptoms of cerebral malaria include, but are not limited to, altered mental status, violent behavior, headache, body ache, and extremely high fever (up to 42°C), followed by coma, metabolic acidosis, hypoglycemia, and possibly seizures and death. Brain swelling, intracranial hypertension, retinal changes (hemorrhages, peripheral and macular whitening, vessel discoloration and/or papilledema) and brainstem signs (abnormalities in posture, pupil size and reaction, ocular movement, or abnormal respiratory patterns) are commonly observed. Without treatment, cerebral malaria is almost always fatal. Despite treatment, mortality caused by cerebral malaria can be as high as 30%, while more than 10% of survivors of the disease can experience short- or long-term neurological complications and cognitive dysfunction.

In some embodiments of the present invention, a treatment for severe malaria is a pre-referral treatment. As used herein, the term *"**pre-referral treatment**"* refers to a treatment that aims to stabilize the patient's condition before transferring them to a higher level of healthcare, such as a health center or hospital, for more advanced care. A pre-referral treatment generally consists of the administration of a single dose of medicine. In the context of severe malaria, it is an essential component of efforts to reduce mortality and improve patient health. Thus, in a pre-referral treatment of severe malaria according to the present invention, a single dose of artesunate powder is administered intranasally to a subject in need thereof before referral of the subject to an appropriate facility for further care.

### 2 - Subjects

As used herein, the term *"**subject**"* refers to a human or another mammal (*e.g*., primate, feline, canine, rodent, bovine, equine, porcine, ovine, and the like), that can develop malaria, but may or may not be suffering from the disease. Non-human subjects may be transgenic or otherwise modified animals. In most embodiments of the present invention, the subject is a human being. In such embodiments, the subject is often referred to as an *"**individual**"* or a *"**patient**".* The terms "individual" and "patient" do not denote a particular age. The term "patient" more specifically refers to an individual suffering from the disease or disorder (here: severe malaria). Thus, the term "severe malaria patient" refers to an individual suffering from severe malaria. A severe malaria patient may or may not have been diagnosed with severe malaria: for example, such a patient may be suspected of suffering from severe malaria, for example in view of symptoms such as those described above.

Preferably, a subject to whom a treatment according to the present invention is to be administered is an individual vulnerable to severe malaria. Individuals vulnerable to severe malaria are generally non-immune subjects or subjects with immature or disturbed immunity.

Thus, in some embodiments, the subject is a child, in particular a child aged 5 or under. Children of that age are vulnerable to severe malaria because of immature immunity - immunity to malaria taking years to develop, increasing with age and exposure. The risk of infection and clinical disease increases after the waning of maternal immunity (at approximately 6 months of age). In the initial years of life, the developing brain is also more susceptible to malaria infection and its after-effects. In certain embodiments, the child has a body weight of less than 20 kg. A treatment according to the present invention is generally intended to be administered to a child 5 years old or younger living in a malaria endemic country. Malaria remains endemic in tropical regions in Africa, Asia, Central and South America, Hispaniola (Dominican Republic and Haiti), the Middle East and Oceania (islands of the Pacific Ocean between Asia and the Americas) (WHO, World Malaria Report 2021, Geneva). The African region carries a disproportionately high share of the global malaria burden, being home to about 94% of all malaria cases and 95% of deaths (of which 80% are deaths of children under 5 years old) (WHO, Malaria. Fact Sheet, 2023). Of the 86 countries that were malaria endemic in 2020, 29 accounted for 95% of malaria cases globally. Four countries accounted for nearly half of all malaria cases worldwide: Nigeria (27%), Democratic Republic of Congo (12%), Uganda (5%) and Mozambique (4%) (WHO, Malaria. Fact Sheet, 2023).

In other embodiments, the subject is a child over 5, a teenager, or a young adult living in a malaria endemic region. It is known that effective population-level interventions against malaria lead to age-shifts, delayed morbidity or rebounds in morbidity and mortality whenever they are deployed in ways that do not permanently interrupt transmission. As the intensity of transmission in endemic areas declines and the development of childhood immunity becomes less likely, the risk of malaria, including severe malaria, in adolescents and young adults increases (Lalloo et al., The Lancet Infect. Dis., 2006, 6(12): 780-793; O'Meara et al., The Lancet, 2008, 372: 1555-1562; Roca-Felter et al., Malaria J., 2010, 9: 282; Carneiro et al., PLOS One, 2010, 5: e8988; Mogeni et al., PLOS Med., 2016, 13: e1002047). Similarly, vaccines, such as the RTS,S/AS01 vaccine and the R21/Matrix-M vaccine recommended by the WHO, provide preerythrocytic malaria immunity, with clinical protection for children up to 5 years of age (RTS,S Clinical Trials Partnership, Lancet, 2015, 386: 31-45). However, this may have the effect of reducing immunity to blood-stage parasites, resulting in prolonged vulnerability and increased incidences of neurological phenotypes in older children and young adults (RTS,S Clinical Trials Partnership, Lancet, 2015, 386: 31-45; Hendriksen et al., J. Infect. Dis., 2023, 207: 351-361).

In other embodiments, a subject to whom a treatment according to the present invention is to be administered is a non-immune child or adult, in particular a pregnant woman, a patient with HIV/AIDS, a non-immune migrant or a non-immune foreign traveler.

For example, the subject may be a pregnant woman. Pregnancy reduces a woman's immunity to malaria, which makes her more susceptible to malaria infection and increases the risk of illness. Furthermore, in pregnant women, malaria is an important cause of stillbirths, infant mortality, miscarriage, and low birth weight, and death, particularly in *P. falciparum* infection.

The non-immune subject may be a HIV/AIDS patient. Opportunistic infections occur more frequently and with greater severity in individuals with compromised immune systems, such as those positive for HIV. HIV can increase parasitic loads in malaria patients and consequently increase malaria transmission rates. Co-infection with *Plasmodium* ssp. and HIV is likely to occur because of the high prevalence of both infections in the same areas, particularly in sub-Saharan Africa. Indeed, with about 14% of the world's population, sub-Saharan Africa is home to roughly 67% of all people living with HIV/AID (UNAIDS: Global AIDS update - Confronting inequalities, 2021; World Population Review: Africa population (1950-2019), 2019).

The non-immune subject may be a non-immune migrant (who travels for employment opportunities, environmental factors, or educational purposes), or a non-immune traveler (who travels for leisure or business, or for national or international army operations). It is widely recognized that human mobility influences the spread of infectious diseases. Travel is a key risk factor for acquiring malaria infection, particularly when travel is to malaria endemic areas or areas of higher transmission intensity than the origin location.

### 3 - Administration

In a treatment according to the present invention, artesunate powder, or an artesunate powder formulation, is administered intranasally to a subject in need thereof. The terms *"**intranasally**", "**via intranasal delivery**"* and *"**via intranasal administration**"* are used herein interchangeably. They refer to a route of administration in which drugs are sprayed or insufflated to one or the two nostrils.

Preferably, artesunate powder, or an artesunate powder formulation, is administered intranasally using a nasal spray device. As used herein, the term *"**nasal spray device**"* refers to any device adapted for spraying a therapeutic agent into a nostril of a human subject, which device is air-tight when assembled. Preferably, the nasal spray device is a unidose or multidose nasal spray device that allows the delivery of a precise dose quickly and easily. In some preferred embodiments, the nasal spray device used in a method of treatment according to the present invention, is a nasal powder spray device. As used herein, the term *"**nasal powder spray device**"* refers to a nasal spray device adapted for the delivery of a therapeutic agent in powder form to the nostril of a human subject. The nasal spray device may be filled immediately prior to artesunate administration (for example about 5 minutes before administration, or about 10 minutes, about 15 minutes, about 20 minutes, about 30 minutes, about 45 minutes or about 1 hour). Alternatively, the nasal spray device or nasal powder spray device is pre-filled and ready-to-used. As used herein, the terms ***"pre-filled and ready-to-use nasal spray device**"* and *"**pre-filled and ready-to-use nasal powder spray device**"* refer to a nasal spray device and a nasal powder spray device having a body part containing the therapeutic agent or formulation thereof and a pump system with a nozzle or other spray function for administration *via* the nasal delivery route, and this device is typically assembled (*i.e*., pre-assembled) and ready for use in a human subject. A pre-filled and ready-to-use nasal (powder) spray device may be reusable and reloaded with a precise amount of artesunate powder, or artesunate powder formulation, according to the dose needed, and available from a different provider. The nasal (powder) spray device containing the therapeutic agent to be administered can be stored for a sufficient period of time, such as 1 month, 3 months, one year or more, and is ready to be used by, for example, a paramedic or the patient.

Examples of nasal spray devices and nasal powder spray devices are known in the art. Examples of nasal powder spray devices that can be used in the context of the present invention include, but are not limited to, the Unidose (UDS) Powder Nasal Spray manufactured by Aptar (Crystal Lake, IL, USA); the Precision Olfactory Delivery (Powder) manufactured by Impel (Seattle, WA, USA); and the UniSpray system manufactured by Nemera (Lyon, France).

The present invention also relates to such a pre-filled, ready-to-use nasal (powder) nasal spray device, as described above, containing artesunate powder, or an artesunate powder formulation, for use in the treatment of severe malaria, in particular in the pre-referral treatment of severe malaria, wherein the dose of artesunate present in the nasal spray device and to be administered intranasally to the subject is lower than the dose of artesunate administered in a standard treatment of severe malaria. Examples of doses of artesunate to be administered intranasally according to a method of the present invention are provided below. The nasal (powder) nasal spray device may be placed in a container comprising a notice or package insert in the form prescribed by a governmental agency regulating the manufacture, use or sale of pharmaceutical or biological products, which notice reflects approval by the agency of manufacture, use or sale for human administration. The notice of package insert may contain instructions for use of the device according to a method of treatment disclosed herein. An identifier, *e.g*., a bar code, radio frequency, ID tags, etc., may be present in or on the kit. The identifier can be used, for example, to uniquely identify the kit for purposes of quality control, inventory control, tracking movement between workstations, etc.

### 4 - Dosage

In a method of treatment of severe malaria according to the present invention, the dose of artesunate administered intranasally to the subject in need thereof is lower than the dose of artesunate administered in a standard treatment of severe malaria, in particular a standard pre-referral treatment of severe malaria. The terms *"**standard treatment**"* and *"**standard treatment of care**"* are used herein interchangeably and refer to a treatment that is accepted by medical experts as a proper treatment for a certain type of disease and that is widely used by health care professionals. In the context of the present invention, a standard treatment for severe malaria is an artesunate-based standard treatment that is recommended by the WHO. There are two artesunate-based standard treatments of severe malaria recommended by the WHO: a first-line treatment, and a pre-referral treatment.

The standard artesunate-based first-line treatment of severe malaria comprises:
- For adults and children weighing 20 kg or more: administration of artesunate at a dose of 2.4 mg/kg body weight by intravenous or intramuscular injection, at 0, 12 and 24 hours, then once daily until oral treatment can be substituted;
- For children weighing less than 20 kg: administration of artesunate at a dose of 3 mg/kg body weight by intravenous or intramuscular injection, at 0, 12 and 24 hours, then once daily until oral treatment can be substituted.

The standard artesunate-based pre-referral treatment of severe malaria comprises:
- Where complete treatment of severe malaria is not possible, but anti-malarial injections are available, adults and children may be given a single dose of artesunate before referral to an appropriate facility for further care;
- When intravenous or intramuscular artesunate injections are not available, children weighing less than 20 kg may receive a rectal administration of artesunate at a dose of 10 mg/kg body weight before referral to an appropriate facility for further care. Generally, for children aged 2 months old to < 3 years old (<_ 10 kg): a suppository containing 100 mg of artesunate is administered rectally, while for children aged 3 years old to < 6 years old (<_ 20 kg): 2 suppositories, each containing 100 mg of artesunate, are administered rectally. It is not recommended to administer rectal artesunate to adult and children weighing 20 kg or more.

Thus, in the context of an artesunate-based standard pre-referral treatment for severe malaria, a child weighing less than 20 kg may receive a single dose of 3 mg/kg body weight of artesunate by injection (preferably intravenous injection) or a single dose of 10 mg/kg body weight of artesunate intrarectally, while an adult or a child weighing 20 kg or more may receive a single dose of 2.4 mg/kg of artesunate by injection (preferably intravenous injection).

In a treatment according to the present invention, in particular in a pre-referral treatment, a dose of artesunate administered intranasally is lower than the dose of artesunate administered in a standard treatment of severe malaria, if said intranasal dose is less than 95% of the dose of artesunate administered in a standard treatment of severe malaria, for example, less than about 90%, less than about 85%, less than about 80%, less than about 75%, less than about 70%, less than about 65%, less than about 60%, less than about 55%, less than about 50%, less than about 45%, less than about 40%, less than about 35%, less than about 30%, less than about 25%, less than about 20%, less than about 15%, less than about 10%, or less than about 5%, of the dose of artesunate administered in a standard treatment of severe malaria.

The terms *"**approximately**"* and *"**about**",* as used herein in reference to a number, generally include numbers that fall within a range of 10% in either direction of the number (greater than or less than the number) unless otherwise stated or otherwise evident from the context (*e.g*., where such number would exceed 100% of a possible value).

In certain embodiments, the dose of artesunate administered intranasally in a method of pre-referral treatment of severe malaria according to the present invention is lower than 10 mg/kg body weight, which is the dose of artesunate administered intrarectally in a pre-referral situation where intramuscular or intravenous injections are not available, *i.e*., generally in a village located far away from a hospital or health center.

In other embodiments, the dose of artesunate administered intranasally in a method of pre-referral treatment of severe malaria according to the present invention is lower than 3 mg/kg body weight, or 2.4 mg/kg body weight, which is the dose of artesunate injected to a subject in need thereof in a situation where intramuscular or intravenous injections are available, *i.e*., in a health center or a medical office.

Thus, a dose of artesunate administered intranasally according to a method of the present invention is preferably lower than 3 mg/kg body weight. The dose of artesunate administered intranasally may be lower than about 2.75 mg/kg body weight, lower than about 2.5 mg/kg body weight, lower than about 2.4 mg/kg body weight, lower than about 2.25 mg/kg body weight, lower than about 2 mg/kg body weight, lower than about 1.9 mg/kg body weight, lower than about 1.8 mg/kg body weight, lower than about 1.7 mg/kg body weight, lower than about 1.6 mg/kg body weight, lower than about 1.5 mg/kg body weight, lower than about 1.4 mg/kg body weight, lower than about 1.3 mg/kg body weight, lower than about 1.2 mg/kg body weight, lower than about 1.1 mg/kg body weight, lower than about 1 mg/kg body weight, lower than about 0.9 mg/kg body weight, lower than about 0.8 mg/kg body weight, lower than about 0.7 mg/kg body weight, lower than about 0.6 mg/kg body weight, or lower than about 0.5 mg/kg body weight. The dose of artesunate administered intranasally may be comprised between about 2 mg/kg body weight and about 0.5 mg/kg body weight, for example between about 1.75 mg/kg body weight and about 0.75 mg/kg body weight, or between about 1.5 mg/kg body weight and 1 mg/kg body weight.

In certain preferred embodiments, in the case of a child of 5 years old or younger (*i.e*., weighing less than 20 kg), in a treatment according to the present invention, in particular in a pre-referral treatment: a dose of artesunate is lower than the dose of artesunate administered by injection in a standard treatment of severe malaria, if said dose is < 3 mg/kg body weight. The dose of artesunate administered intranasally may be a dose of less than 2.75 mg/kg body weight, or less than 2.5 mg/kg body weight, or less than 2 mg/kg body weight, or less than 1.5 mg/kg body weight, or less than 1 mg/kg body weight, or less than 0.5 mg/kg body weight. For example, the dose of artesunate administered intranasally may be 2.75 mg/kg body weight, 2.7 mg/kg body weight, 2.6 mg/kg body weight, 2.5 mg/kg body weight, 2.4 mg/kg body weight, 2.3 mg/kg body weight, 2.2 mg/kg body weight, 2.1 mg/kg body weight, 2.0 mg/kg body weight, 1.9 mg/kg body weight, 1.8 mg/kg body weight, 1.7 mg/kg body weight, 1.6 mg/kg body weight, 1.5 mg/kg body weight; 1.4 mg/kg body weight, 1.3 mg/kg body weight, 1.2 mg/kg body weight, 1.1 mg/kg body weight, 1 mg/kg body weight, 0.9 mg/kg body weight, 0.8 mg/kg body weight, 0.7 mg/kg body weight, 0.6 mg/kg body weight, 0.5 mg/kg body weight, or less than 0.5 mg/kg body weight.

In the case of an adult or child of over 5 years old (*i.e*., weighing 20 kg or more), in a treatment according to the present invention, in particular in a pre-referral treatment: a dose of artesunate is lower than the dose of artesunate administered by injection in a standard treatment of severe malaria, if said dose is < 2.4 mg/kg body weight. The dose of artesunate administered intranasally may be a dose of less than 2.2 mg/kg body weight, or less than 2.1 mg/kg body weight, or less than 2 mg/kg body weight, or less than 1.5 mg/kg body weight, or less than 1 mg/kg body weight, or less than 0.5 mg/kg body weight. For example, a dose of artesunate lower than the dose of artesunate administered intramuscularly in a standard treatment of severe malaria may be 2.2 mg/kg body weight, 2.1 mg/kg body weight, 2.0 mg/kg body weight, 1.9 mg/kg body weight, 1.8 mg/kg body weight, 1.7 mg/kg body weight, 1.6 mg/kg body weight, 1.5 mg/kg body weight; 1.4 mg/kg body weight, 1.3 mg/kg body weight, 1.2 mg/kg body weight, 1.1 mg/kg body weight, 1 mg/kg body weight, 0.9 mg/kg body weight, 0.8 mg/kg body weight, 0.7 mg/kg body weight, 0.6 mg/kg body weight, 0.5 mg/kg body weight, or less than 0.5 mg/kg body weight.

The invention will be further illustrated by the following examples. However, these examples and associated figures should not be interpreted in any way as limiting the scope of the present invention.

### Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually carried out or data were actually obtained.

### Example: First-in-Human, Single Center, Open-Label, Dose-Escalation Phase I Study in Healthy Non-Malaria Adults to Evaluate the Safety and Pharmacokinetic Parameters of Single Dose Intranasal Spraying of Artesunate Powder

### 1 - Objectives

The primary objective is to assess the pharmacokinetic parameters of intranasal administration of artesunate powder, the endpoints being: artesunate and dihydroartemisinin Cₘₐₓ (maximal concentration), Tₘₐₓ (time to peak drug concentration) and AUC₀₋₂₄ₕ (area under the curve from time 0 to 24h). The secondary objective is to evaluate safety and tolerability of intranasal administration of artesunate powder, the endpoints being adverse events/severe adverse events (AE/SAE) incidence and severity, hematology tests, and electrocardiogram abnormalities.

### 2 - Study Design

The phase I study aims to determine the safety and pharmacokinetic (PK) parameters of intranasal administration of artesunate powder in healthy non-malaria adult volunteers.
***1. Dosing Regimens, Artesunate Powder and Intranasal Administration.*** Three dosing regiments will be assessed in 3 groups of 4 adults: 0.5 mg/kg body weight artesunate powder per dose, 1 mg/kg body weight artesunate powder per dose, and 2.4 mg/kg body weight artesunate powder per dose.

A nasal spray device will be used to deliver a single precise dose of artesunate powder quickly and easily. Nasal powder formulation offers unique benefits to both product owners and patients including greater product stability, enhanced bioavailability, higher dosing and delivery of molecules with low aqueous solubility. The nasal delivery device will be filled using good distribution practice (GDP) for active substances with doses ranging from 10 to 80 mg by the manufacturer and provided ready-to-use to the study site. When the dose needed will exceed 80 mg according to the volunteer body weight, a second nasal spray device will be used immediately in the second nostril. One device per nostril will be manually actuated with a fixed insertion depth of 1.5 cm, delivery angle (horizontal plane) of 45° and angle from the center wall of 0 to 5°.

Artesunate powder at the lowest dose (0.5 mg/kg body weight) will be administered to volunteers of a first sub-group of the first dose group; undesirable effects will be evaluated 24 hours following treatment; and artesunate powder will be administered to volunteers of a second sub-group of the first group at the same lowest dose, and undesirable effects will be evaluated 24 hours following treatment. After agreement from the Safety Monitoring Committee (SMC), the same will performed with the second group of volunteers at a dose of 1 mg/kg body weight artesunate powder per dose, and then with the third group of volunteers at a dose of 2.4 mg/kg body weight artesunate powder per dose.

***2. Study Subjects.*** Study subjects will be healthy, non-malaria adult volunteer males or non-pregnant, non-lactating females aged 18-25 years (inclusive) from the Bamako (Mali) area. To be eligible for inclusion, participants will have to be able to comply with study procedures and be in generally good health. They will be assessed for serious acute or chronic illnesses by screening laboratory tests, complete medical history, physical and nasal examination. Potential participants will be excluded if they are immunosuppressed, or have a history of recent malaria.

***3. Inclusion Criteria.*** Potential subjects will be required to meet all of the following criteria for enrollment in the study:
- Healthy adults between the ages of 18-25 (inclusive);
- Able and willing to provide written, informed consent;
- Able and willing to comply with all research requirements, in the opinion of the Investigator;
- Axillary temperature < 37.5°C;
- Laboratory Criteria within 10 days before enrollment:
- Hemoglobin ≥ 11.7 g/dL for women; ≥ 12.0 g/dL for men;
- White Blood Cell count: 3,800-10,800 cells/mm³;
- Platelets > 140,000/mm³.

***4. Exclusion Criteria.*** Subjects meeting any of the following criteria will not be eligible for inclusion in the present study:
- Ages < 18 or < 25;
- Pregnant or lactating female;
- Not able or not willing to provide written, informed consent;
- Signs and symptoms of severe or non-severe malaria according to WHO 2015 criteria;
- Active infections including tuberculosis;
- Axillary temperature ≥ 37.5°C;
- Any confirmed or suspected immunosuppressive or immunodeficient condition, including human immunodeficiency virus (HIV) infection;
- Severe malnutrition (body mass index (BMI) less than 70% of median normalized WHO reference weight);
- Severe vomiting (defined as more than 3 times in the 24 hours prior to inclusion in the study) or severe diarrhea (defined as more than 3 watery stools in the 24 hours prior to inclusion in the study);
- Anemia (Hemoglobin level < 11.7 g/dL);
- History of malignancy of any organ system (other than localized basal cell carcinoma of the skin or *in situ* cervical cancer), treated or untreated, within the past 5 years, regardless of whether there is evidence of local recurrence or metastases;
- Known chronic underlying disease such as sickle cell disease, and severe cardiac, renal or hepatic impairment;
- Known active or uncontrolled ear, nose and throat (ENT) disease;
- Inability to tolerate oral medicine (in tablet and/or liquid form);
- Patients with prior antimalarial therapy within minimum of 4 weeks;
- Use of other investigational drugs within 5 half-lives of enrollment, or within 30 days or until the expected pharmacodynamic effect has returned to baseline, whichever is longer;
- History or family history of long QT syndrome or sudden cardiac death, or any other clinical condition known to prolong the QTc interval, such as history of symptomatic cardiac arrhythmias, clinically relevant bradycardia or severe heart disease;
- History of hypersensitivity to artesunate and dihydroartemisinin, or its excipient, or to drugs of similar chemical classes.

### 3 - Assessment Schedule

Volunteers will be screened for eligibility prior to inclusion one day before the start of the study. After inclusion, volunteers will be randomized to the three dose groups (0.5 mg/kg body weight, 1 mg/kg body weight and 2.4 mg/kg body wight), and nasal delivery devices loaded with artesunate powder will be selected according to the volunteers' body weight. In case of required dose exceeding 100 mg, the dose will be split into two devices, one for each nostril, and administered in a row.

### 1. Assessment Schedule Table.

The assessment schedule is presented in the following table.

| **Period** | **Screening** | **Inclusion** | **Treatment** | **Clinical and blood tests** | | | | | | | | | | **Follow-up** |
|---|---|---|---|---|---|---|---|---|---|---|---|---|---|---|
| **Days** | **0** | **0** | **Day 1** | | | | | | | | | | | **Day 2** |
| **Visit Name** | **Screening** | **Inclusion** | | | | | | | | | | | | |
| Demography | X | | | | | | | | | | | | | |
| Informed consent | X | | | | | | | | | | | | | |
| Inclusion / Exclusion criteria | | X | | | | | | | | | | | | |

| **Days** | **0** | **0** | **Day 1** | | | | | | | | | | | **Day 2** |
|---|---|---|---|---|---|---|---|---|---|---|---|---|---|---|
| | | | **Minutes** | | | | | **Hours** | | | | | | **Hours** |
| **Intranasal treatment** | | | **X** | | | | | | | | | | | |
| **Time (post-dose)** | **-24 hours** | | **0** | **0** | **5** | **20** | **40** | | **1** | **2** | **4** | **6** | **8** | **24** |
| Blood sampling | | 20G Peripheral Venous Catheter (PVC) | | | | | | | | | | | | Direct venous puncture (vacutainer) |
| Malaria blood film for asexual parasite | x | | | | | | | | | | | | | |
| Adverse Events | | | X | X | | | | X | | | | | | X |
| Medical history/current medical conditions | X | | | | | | | | | | | | | |
| Physical Examination | X | | | X | | | | | X | | | X | | x |
| Body Height | X | | | | | | | | | | | | | |
| Body Weight | X | | | X | | | | | | | | | | X |
| Blood Pressure | X | | | X | | | | | X | | | X | | X |
| Body Temperature | X | | | X | | | | | X | | | X | | X |
| Pulse rate | X | | | X | | | | | X | | | X | | X |
| Chemistry | X | | | | | | | | | | | X | | X |
| Hematology | X | | | | | | | | | | | X | | X |

***2. Blood Sampling.*** Included volunteers will be equipped with a 20G peripheral venous catheter (PCV) to facilitate blood sampling and reduce pain and discomfort for the first 8 hours:
- Whole blood will be collected immediately after injection, and at 5, 20, 40 minutes, and 1, 2, 4, 6, 8 hours using the PCV.
- The blood sampling method will be identical for all participants: PVC will be washed with 2 mL saline, with a 20-second wait period, followed by vein blocking for 15 seconds; a 5-mL syringe will be attached to the PCV; a new 1.0-mL syringe will be attached for drawing 1.0 mL of blood (Ben Shabat et al., Medicine, 2022, 101: e29268).
- A total of 5 mL of blood will be collected during the first hours of post-treatment, and 5 mL more between H1 and H8.
Samples for pharmacokinetic study will be immediately frozen at -80°C on site. Then the peripheral venous catheter will be extracted, and the local skin will be carefully examined.

A standard 23G needle attached to a vacutainer will be used for sampling blood *via* direct venous sampling (DVS) from the opposite limb as that with the PVC. A total of 5 mL will be collected by DVS at Day 2. Samples for chemical and hematological tests will be sent immediately to a dedicated medical laboratory.

Standard physical examination including nasal cavities will be performed by a medical doctor and an ENT specialist to observe nasal mucosa, turbinates, nasal floor and to detect epithelial breach or inflammatory reaction.

### 4 - Ethical and Regulatory Considerations

The study will be conducted in accordance with the international and national applicable regulations and laws in the clinical trial conduct. The trial will be conducted in accordance with the law No. 09-059 of December 18, 2009, regulating biomedical research on humans in Mali. The study protocol and associated informed consent documents will be reviewed by the ethics committee of the Université des Sciences, Techniques et Technologie (USTTB) de Bamako. Formal approval from the Ethics Committee of the USTTB will be required prior to the recruitment, screening, and enrolment of subj ects. Any amendments to the protocol or informed consent documents will have to be approved by the Ethics Committee prior to implementation. The Principal Investigator (PI) will notify the Ethics Committee of deviations from the protocol and reportable SAEs, as per applicable according the USTTB Ethics Committee policy.
***1. Informed Consent Process.*** The Investigator or designee will obtain a subject's informed consent in accordance with the requirements of ICH E6 GCP before any study procedures or data collection are performed. In addition to oral explanations regarding the study, all participants will be provided with a Participant Information Sheet and Informed Consent Form containing relevant information. The volunteers will be given an opportunity to ask questions and allowed sufficient time to consider participation in the research trial. The Informed Consent Form will have to be signed prior to starting any study procedure.
***2. Privacy and Confidentiality of Personal Data.*** The privacy and confidentiality of personal data will be managed in accordance with the law No. 2013-015 of May 21, 2013, regulating the protection of personal data in Mali (webpage: apdp.ml).

*Study Discontinuation and Disclosure.* This study may be prematurely terminated if there is sufficient reasonable cause, including, but not limited to:
- Determination of unexpected, significant, or unacceptable risk to volunteers;
- Results of interim analysis;
- Insufficient compliance to protocol requirements;
- Regulatory/ethical authorities' request.

### 5 - Safety Oversight

Safety oversight will be under the direction of the Safety Monitory Committee (SMC) which will review adverse effects (AE) data from the first group of 4 participants with 0.5 mg/kg body weight of artesunate powder per dose and to ensure that no halting rules have been met prior to the progression to the subsequent dose group of 1 mg/kg body weight artesunate powder per dose and 2.4 mg/kg body weight artesunate powder per dose.

### 6 - Quality Assurance and Control

The clinical site will perform internal quality management of study conduct, data and biological specimen collection, documentation, and completion. The study site quality management plan will be adapted to fit the need of the study. The clinical trial will be conducted by the MRTC Bandiagara Malaria Program (BMP), which as conducted clinical trials under ICH-GCPs since 2003. The BMP was acknowledged by African Network for Drugs Innovation (ANDI) as a Center of Excellence for Clinical Development of Malaria Product in 2011. The MRTC Clinical laboratory that will undertake the hematology and blood chemistry analyses is CAP accredited since 2011.

### 7 - Protocol Deviation

A protocol deviation is any noncompliance with the clinical trial protocol, International Conference on Harmonization Good Clinical Practice (ICH GCP), or Pharmacy Manual requirements. The noncompliance may be either on the part of the participant, the investigator, or the study site staff. As a result of deviations, corrective actions are to be developed by the site and implemented promptly. It is the responsibility of the Principal Investigator and study staff to use continuous vigilance to identify and report deviations within 5 working days of identification of the protocol deviation. Protocol deviations must be sent to the EC per their guidelines.

## Claims

1. Artesunate for use in the treatment of severe malaria in a subject, wherein artesunate is administered as a powder to the subject in need thereof *via* intranasal delivery and wherein artesunate is administered to the subject at a dose which is lower than the dose of artesunate administered in a standard treatment of severe malaria.

2. Artesunate for the use according to claim 1, wherein the treatment of severe malaria is a pre-referral treatment.

3. Artesunate for the use according to claim 1 or claim 2, wherein treatment of severe malaria comprises prevention of at least one complication of severe malaria, in particular cerebral malaria.

4. Artesunate for the use according to any one of claims 1 to 3, wherein the subject is a child, in particular a child of 5 years of age or younger living in a malaria endemic country.

5. Artesunate for the use according to any one of claims 1 to 3, wherein the subject is a child over 5 years old, an adolescent or a young adult living in a malaria endemic country.

6. Artesunate for the use according to any one of claims 1 to 3, wherein the subject is a non-immune child or adult, in particular a pregnant woman, a patient with HIV/AIDS, a non-immune migrant or a non-immune foreign traveler.

7. Artesunate for the use according to any one of claims 1 to 6, wherein said intranasal delivery is intranasal spraying.

8. Artesunate for the use according to claim 7, wherein artesunate intranasal spraying is performed using a nasal spray device, wherein the nasal spray device is a pre-filled, ready-to-use nasal spray device or wherein the nasal spray device is filled immediately prior to artesunate administration.

9. Artesunate for the use according to any one of claims 1 to 8, wherein artesunate is administered in a single dose.

10. Artesunate for the use according to any one of claims 1 to 9, wherein the standard treatment of severe malaria is a first-line treatment of severe malaria and comprises:
a) administration of artesunate at a dose of 2.4 mg/kg body weight by intravenous or intramuscular injection to an adult or a child weighing 20 kg or more; or
b) administration of artesunate at a dose of 3 mg/kg body weight by intravenous or intramuscular injection to a child weighing less than 20kg.

11. Artesunate for the use according to any one of claims 1 to 9, wherein the standard treatment of severe malaria is a pre-referral treatment of severe malaria and comprises:
a) administration of artesunate at a dose of 2.4 mg/kg body weight by injection to an adult or a child weighing 20 kg or more; or
b) administration of artesunate at a dose of 10 mg/kg body weight by rectal administration to a child weighing less than 20kg.

12. Artesunate for the use according to any one of claims 1 to 11, wherein the dose of artesunate administered *via* intranasal delivery is lower than 3 mg/kg body weight.

13. Artesunate for the use according to claim 12, wherein the dose of artesunate administered *via* intranasal delivery is comprised between about 2 mg/kg body weight and about 0.5 mg/kg body weight, in particular comprised between about 1.75 mg/kg body weight and about 0.75 mg/kg body weight, or between about 1.5 mg/kg body weight and about 1 mg/kg body weight.

14. A pre-filled, ready-to-use nasal spray device containing artesunate powder for use in the pre-referral treatment of severe malaria in subject, wherein artesunate present in the nasal spray device is in powder form and is administered to the subject at a dose which is lower than the dose of artesunate administered in a standard treatment of severe malaria.

15. The pre-filled, ready-to-use nasal spray device for the use according to claim 14, wherein the nasal spray device comprises a dose of artesunate which is lower than 3 mg/kg body weight.
